# EUROPÄISCHE PATENTSCHRIFT

(11) **EP 3 433 776 B1**
(45) Veröffentlichungstag und Bekanntmachung des Hinweises auf die Patenterteilung: **16.03.2022**
(21) Anmeldenummer: 17712112.6
(22) Anmeldetag: 20.03.2017
(51) Int. Cl.: G16H 40/63, G16H 40/67, A61M 1/14

(54) **ENTFERNTES STEUERN VON MELDUNGEN FÜR EIN DIALYSEGERÄT**
REMOTE PROCESSING OF MESSAGES FOR A DIALYSIS APPARATUS
COMMANDE À DISTANCE DES MESSAGES D'UN APPAREIL DE DIALYSE

(30) Priorität: 21.03.2016 DE 102016105199
(43) Veröffentlichungstag der Anmeldung: 30.01.2019
(73) Patentinhaber: Fresenius Medical Care Deutschland GmbH, 61352 Bad Homburg (DE)
(72) Erfinder: MAIERHOFER, Andreas, 97422 Schweinfurt (DE); MUELLER, Carsten, 97502 Euerbach (DE)
(74) Vertreter: Schwarz, Claudia
(86) Internationale Anmeldenummer: PCT/EP2017/056535
(87) Internationale Veröffentlichungsnummer: WO 2017/162576

(56) Entgegenhaltungen:
- EP-A1- 1 480 180
- DE-A1-102007 002 305
- DE-A1-102011 083 957
- DE-A1-102011 107 795
- US-A1- 2013 131 845
- US-A1- 2014 113 553

## Beschreibung

Die vorliegende Erfindung liegt auf den Gebieten der Dialysegeräte und der elektronischen Steuerungen und betrifft insbesondere ein Verfahren und eine Fernsteuereinheit sowie ein Anwendungsgerät mit einer solchen Fernsteuereinheit zum entfernten Steuern und Bearbeiten von Meldungen für ein Dialysegerät von einem entfernten Anwendungsgerät.

Bekannte, moderne medizintechnische Geräte, wie z.B. Dialysegeräte, umfassen eine Ein- und Ausgabeeinheit zur Ein- und Ausgabe von Daten, die häufig als berührungssensitiver Bildschirm zur Steuerung des Gerätes ausgebildet sein kann, wie beispielsweise das Hämodialysegerät 5008 von Fresenius Medical Care. Für die Bedienoberflächen der Dialysegeräte wird üblicherweise eine kapazitive Sensortechnik verwendet. Ein Touchscreen mit einer kapazitiven Sensortechnik ist in der DE10 2011 011 769 A1 näher beschrieben, bei der eine zusätzliche Piezoelementschicht mit Piezoelementen verwendet wird, um taktiles Feedback zu detektieren.

Die Dialysegeräte, insbesondere deren Displays zur Bedienung und Steuerung des Gerätes, werden in der klinischen Praxis in der Regel von mehreren Personen bedient oder von einer Person, die jedoch in Sequenz mehrere medizinische und möglicherweise kontaminierte Geräte oder Patienten bedient und zwischen den Bedienungen somit stets in zeitaufwändiger Weise gefordert ist, eine Desinfektion auszuführen, damit es nicht über die Berührung des Bildschirms zu ungewollten Verunreinigungen kommt, indem z.B. pathogene Keime oder Mikroorganismen eine Kreuzkontamination verursachen, also eine direkte oder indirekte Übertragung von potentiell schädlichen Substanzen.

Um eine solche Übertragung von Krankheitserregern und Verunreinigungen zu vermeiden, ist es im Stand der Technik bekannt, vor und/oder nach jeder Bedienung bzw. Berührung der Benutzeroberfläche des Dialysegerätes routinemäßig eine Desinfektion auszuführen und insbesondere dann, wenn der Anwender (der Arzt oder die Krankenschwester) an einem anderen Anwendungsgerät arbeitet (z.B. an einem anderen Dialysegerät) oder in direktem Patientenkontakt ist. Dazu muss der Anwender gegebenenfalls zunächst seine Hände und/oder die Benutzeroberfläche des Dialysegeräts desinfizieren. Erst danach kann er die Benutzeroberfläche des Dialysegerätes bedienen. Da dieses Vorgehen sehr zeitaufwändig und umständlich ist, besteht die Gefahr, dass es in der therapeutischen Praxis unterlassen wird, was die Gefahr von Kreuzkontaminationen steigert.

Die DE 10 2011 107 795 A1 der Anmelderin zeigt ein System zur entfernten Steuerung von Dialysegeräten durch ein entferntes Steuergerät, das sich in einen Kontrollraum befindet.

Die US 2013/0133036 A1 offenbart ein System zum Fernzugriff auf ein Dialysegerät, um z.B. dem Arzt oder der behandelnden Person einen Zugriff auf das Dialysegerät zu ermöglichen, das sich entfernt, z.B. im privaten Umfeld des Patienten befinden kann, ohne, dass der Arzt sich zum Dialysegerät im Hause des Patienten bewegen muss.

Das Problem bei den bekannten Geräten aus dem Stand der Technik ist darin zu sehen, dass dem Sicherheitsaspekt nicht in ausreichendem Maße Rechnung getragen wird. Bei dem Quittieren von Meldungen, wie z.B. Alarmmeldungen, die auf dem und für das medizinische Gerät ausgegeben werden, muss sichergestellt werden, dass die behandelnde Person, die an einem anderen Anwendungsgerät arbeitet und sozusagen von entfernt ein Quittieren der Meldung veranlassen möchte, auch Sichtkontakt zu dem Patienten des medizinischen Gerätes hat, um Fehlbedienungen zu vermeiden.

Die vorliegende Erfindung hat sich deshalb zur Aufgabe gestellt, das Steuern eines medizinischen Gerätes und insbesondere das Bearbeiten von Meldungen für das medizinische Gerät, insbesondere für eine Dialysemaschine, von einem entfernten Anwendungsgerät zu verbessern. Insbesondere soll die Sicherheit bei der Bedienung von Dialysemaschinen erhöht und die Gefahr von Kreuzkontaminationen verringert werden. Ferner soll der Bedienprozess im Rahmen einer Dialyse flexibler und zugleich effizienter gestaltet werden.

Diese Aufgabe wird erfindungsgemäß durch die Merkmale gemäß den beiliegenden nebengeordneten Patentansprüchen gelöst.

Im Folgenden wird die Erfindung anhand der verfahrensgemäßen Aufgabenlösung beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Ansprüche (die beispielsweise auf ein System oder auf ein Produkt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch elektronische Hardware-Module oder Mikroprozessor-Module, des Systems bzw. des Produktes ausgebildet und umgekehrt.

Das erfindungsgemäße Verfahren ist für eine Ein- und Ausgabeeinheit, insbesondere für einen Touchscreen, eines medizinischen Gerätes, insbesondere eines Dialysegerätes, ausgelegt. Das Verfahren kennzeichnet sich dadurch, dass ein entferntes Bearbeiten einer Meldung nur auf solchen entfernten Anwendungsgeräten aus möglich ist, die ein Sicherheitskriterium erfüllen.

Gemäß einem Aspekt betrifft die Erfindung somit ein Verfahren nach Anspruch 1.

Der Begriff Fernsteuern ist im Rahmen dieser Anmeldung einschränkend zu verstehen und betrifft das Bearbeiten von Meldungen von oder für ein medizinisches Gerät auf einem entfernten Anwendungsgerät. Das entfernte Bearbeiten kann die Eingabe und das Erfassen eines Quittierungssignals (Bearbeitungssignal) umfassen. Das Bearbeitungssignal wird üblicherweise auf einer Benutzeroberfläche des Anwendungsgerätes eingegeben. Alternativ kann es sich aber auch um eine Texteingabe, eine Bewegungsgeste oder um ein Sprachsignal handeln. Das entfernte Bearbeiten der Meldungen umfasst eine Eingabe von Bearbeitungssignalen seitens des Anwenders auf dem Anwendungsgerät. Bei den Bearbeitungssignalen kann es sich um eine Quittierung einer Meldung oder um einen oder mehrere Bearbeitungsbefehl(e) handeln, die auf dem medizinischen Gerät ausgeführt werden sollen. Es ist ebenso möglich, dass das Bearbeitunassignal kein binäres Signal zur Bestätigung ist, sondern die Eingabe von Werten umfasst. Das entfernte Bearbeiten kann somit ein Erfassen von zumindest einem Bearbeitungssignal und das Senden des erfassten Bearbeitungssignals an das medizinische Gerät umfassen.

Mit der erfindungsgemäßen Lösung wird eine Option zur Fernsteuerung des medizinischen Gerätes bereitgestellt. Dafür muss eine Fernbearbeitungsprozedur (im Folgenden auch kurz Bearbeitungsprozedur) auf dem jeweiligen Anwendungsgerät implementiert und initialisiert werden. Nur dann kann eine sichere Fernbearbeitung von Meldungen für das Dialysegerät von dem Anwendungsgerät aus durchgeführt werden. Es liegt aber ebenso im Rahmen der Erfindung, dass diese Option zur Fernbearbeitung von Meldungen nicht genutzt wird, sondern dass dennoch eine direkte Bearbeitung der Meldung auf dem die Meldung sendenden Dialysegerät erfolgt. Ebenso liegt es im Rahmen der Erfindung, dass die Fernbearbeitungsoption genutzt wird und eine indirekte Bearbeitung der Meldung auf einem entfernten Gerät, nämlich auf dem Anwendungsgerät, erfolgt. So wird zwischen dem Bereitstellen der Bearbeitungsprozedur auf dem Anwendungsgerät (also dessen Installation auf dem Rechnersystem des Anwendungsgerätes) und dem Ausführen (Ablauf der Applikation bzw. der Anwendung) derselben differenziert. Nach dem Bereitstellen der Bearbeitungsprozedur muss diese, wie oben beschrieben, nicht zwangsläufig ausgeführt werden. Dies erfolgt ohnehin nur nach Überprüfung des Sicherheitskriteriums. Die Bearbeitungsprozedur umfasst einen Signalaustausch zwischen dem Anwendungsgerät und dem fernzubedienenden, medizinischen Gerät. Dieser Signalaustausch kann digitale und/oder analoge Signale umfassen. Die Bearbeitungsprozedur stellt insbesondere eine Benutzeroberfläche oder Ausschnitte derselben auf dem Anwendungsgerät bereit, die entsprechende Steuerfelder umfassen und stellvertretend für die Steuerfelder auf dem medizinischen Gerät erfindungsgemäß auf dem Anwendungsgerät bedient werden können. Es ist auch möglich, die gesamte Benutzeroberfläche des medizinischen Gerätes auf dem Anwendungsgerät abzubilden oder nur aktuell zu bedienende Steuerfelder. Damit wird es möglich, Meldungen, die auf dem medizinischen Gerät generiert werden, auf dem Anwendungsgerät über die Bedienung eines entsprechenden Steuerfeldes zu quittieren.

Erfindungsgemäß kann jedoch die Sicherheit in der Bedienung erhöht werden, indem ein Missbrauch durch eine ungeprüfte Bedienung bzw. Aufschaltung von einem anderen Gerät aus zuverlässig vermieden werden kann. Dazu wird ein zusätzliches Bauteil bereitgestellt, die Identifikationsvorrichtung, die dazu bestimmt ist, ein Sicherheitskriterium zu überprüfen. Sie kann insbesondere dazu ausgelegt sein, automatisch zu überprüfen, ob ein Sichtkontakt zwischen der Bedienperson am Anwendungsgerät und dem Patienten am medizinischen Gerät oder mit dem medizinischen Gerät an sich besteht.

Im Folgenden wird die Erfindung für ein Dialysegerät als Beispiel eines medizinischen Gerätes beschrieben, z.B. eines Hämodialysegerätes oder eines Peritonealdialysegerätes. Für den Fachmann liegt es jedoch auf der Hand, dass die Erfindung ebenso auf andere medizintechnische, computergesteuerte Geräte oder (Fluidmanagement-) Maschinen oder Blutbehandlungsgeräte angewendet oder übertragen werden kann, die über einen Touchscreen gesteuert werden.

Das medizinische Gerät kann unterschiedliche Rollen einnehmen: nämlich als fern zu bedienendes Gerät oder als Anwendungsgerät. Als Anwendungsgerät wird ein Gerät dann bezeichnet, wenn ein Anwender sich gerade vor dem Anwendungsgerät befindet und z.B. dort einen Patienten behandelt oder andere Tätigkeiten ausführt. Das Anwendungsgerät muss nicht zwingend ein medizinisches Gerät sein; es kann sich auch um einen zentralen Server, ein mobiles Kommunikationsgerät oder um einen Verwaltungs- oder Bildbearbeitungsrechner handeln.

Bei dem Display handelt es sich in einer bevorzugten Ausführungsform der Erfindung um einen kapazitiven Touchscreen mit Multi-Sensor-Funktionalität, der auch gleichzeitige Berührungen detektieren kann. Ein Beispiel eines solchen Touchscreens ist in der DE10 2011 011 769 A1 näher beschrieben. Das Display stellt eine Benutzeroberfläche (auch Monitor genannt) dar und dient zur Erfassung der Eingabesignale (z.B. auf Eingabefeldern) zur Steuerung und Bedienung des Anwendungsgerätes. Das Display umfasst Interaktionsflächen, Schaltelemente, Kontrollfelder etc. zur Steuerung des Anwendungsgerätes. Erfindungsgemäß umfasst das Display jedoch zusätzlich noch einen Fernbearbeitungsbereich, der für die Fernbearbeitung eines entfernten Dialysegerätes reserviert ist. Dies kann beispielsweise durch die Einblendung eines zusätzlichen Fensters umgesetzt werden.

Das Aktivierungssignal dient zur Initiierung der Fernbearbeitung von Meldungen. Vorzugsweise wird das Aktivierungssignal nur an ausgewählte Anwendungsgeräte gesendet, nämlich an Ziel-Anwendungsgeräte, die in einer Definitionsphase konfiguriert worden sind. Dabei kann es konfiguriert werden, welche Ziel-Anwendungsgeräte grundsätzlich für ein bestimmtes medizinisches Gerät in Frage kommen. Dabei kann eine Zulässigkeitsbedingung ausgewertet werden, die z.B. prüft, ob sich das fern zu bedienende medizinische Gerät und das Anwendungsgerät im selben Raum befinden. Wahlweise können noch weitere Bedingungen definiert werden. Dies erfolgt vorzugsweis menügesteuert in der Definitionsphase. Der Anwender hat jedoch auch die Möglichkeit, eine Auswahl zu treffen oder bestimmte Bedingungen und/oder Ziel-Anwendungsgeräte zu definieren. Je nach Ausführungsform der Erfindung kann nach Erfassen eines Aktivierungssignals auf einem Anwendungsgerät, das die Zulässigkeitsbedingung erfüllt, und bei positiver Überprüfung des Sicherheitskriteriums entweder eine Meldung fernbedient werden oder eine Abfolge von Meldungen. Dies kann auch in dem Aktivierungssignal spezifiziert werden (z.B. über das Setzen eines Bits für die Gültigkeit der Fernbedienungsoption).

Bei der Meldung handelt es um eine Statusmeldung in Bezug auf das medizinische Gerät, die in unterschiedlichen Sicherheitsstufen klassifiziert werden. Die unterschiedlichen Sicherheitsstufen können ebenfalls in der Definitionsphase konfiguriert werden. Die Meldungen erfordern zumindest die Eingabe eines Bestätigungs- oder Bearbeitungssignals zum Quittieren der Meldung durch einen Anwender. Die Meldungen können in unterschiedlichen Formaten ausgegeben werden (z.B. optisch und/oder akustisch etc.). Die Meldung kann z.B. als Alarm ausgebildet sein. Die Meldungen können in einem unterschiedlichen Modus vorliegen. Sie können unterschiedliche Sicherheitsaspekte des Gerätebetriebs betreffen und ein unterschiedliches Risikopotential haben. Je nach Modus der Meldung wird eine unterschiedliche Bearbeitung der Meldung gefordert. So kann eine Alarmmeldung, die auf eine Gesundheitsgefährdung des Patienten hinweist, eine umgehende und hoch priorisierte Bearbeitung erfordern im Vergleich zu einer Statusmeldung zum Batteriestatus eines Ersatzmoduls.

Das Bearbeitungssignal kann eine Eingabe auf einem berührungssensitiven Display, eine Texteingabe, eine Auswahl in einem Menü oder eine akustische oder optische Eingabe umfassen, z.B. in Form einer Geste. Die Fernbedienung bzw. das entfernte Bearbeiten umfasst somit die Eingabe eines Bearbeitungs- oder Bestätigungssignals, z.B. für eine Alarmmeldung, die Eingabe eines Anwendersignals (Tastendruck) auf einem Bedienfeld des Displays auf dem entfernten Anwendungsgerät. Das Bearbeitungssignal ist eineindeutig einer Meldung zugeordnet. Das Bearbeitungssignal wird erfindungsgemäß auf einem anderen Gerät erfasst als das Gerät, auf dem die Meldung erzeugt worden ist.

Das Sicherheitskriterium dient zur Überprüfung, ob das jeweilige Anwendungsgerät zur Fernbearbeitung der jeweiligen Meldung unter Berücksichtigung der jeweiligen Sicherheitsstufe der Meldung für das jeweilige medizinische Gerät zugelassen ist. Die Überprüfung umfasst insbesondere, ob ein Sichtkontakt zwischen dem Anwender des Anwendungsgerätes und dem fern zu bedienenden medizinischen Gerät oder dessen Patienten besteht. Dazu wird ein Sichtkontaktsignal ausgewertet.

Das Sichtkontaktsignal repräsentiert einen Zustand von dem Anwender des Anwendungsgerätes und dem jeweiligen medizinischen Gerät (oder seinem Patienten). Insbesondere wird mit dem Sichtkontaktsignal automatisch erfasst, ob ein Sichtkontakt zwischen dem Anwender des Anwendungsgerätes und dem medizinischen Gerät und/oder dem am medizinischen Gerät angeschlossenen Patienten besteht. Gemäß einer ersten Ausführungsform der Erfindung wird durch das Sichtkontaktsignal spezifiziert, ob ein Sichtkontakt prinzipiell bestehen kann und gemäß einer zweiten Ausführungsform der Erfindung, ob ein Sichtkontakt aktuell besteht. In einer bevorzugten Ausführungsform der Erfindung wird für jede Meldung ein Sichtkontaktsignal angefordert, das dann jeweils der Meldung zugeordnet wird. Wie bereits erwähnt, können in einer anderen Ausführungsform der Erfindung auch mehrere Meldungen (z.B. eine Folge von Meldungen) durch das Sichtkontaktsignal der ersten Meldung bearbeitet werden.

Gemäß einer vorteilhaften Ausführungsform der Erfindung wird das Sicherheitskriterium in einer Definitionsphase konfiguriert, die der eigentlichen Fernsteuerphase zeitlich vorgelagert ist. Damit ist eine Vorkonfiguration möglich, indem dem Anwender auf einer Benutzeroberfläche Voreinstellungen für die Konfiguration des Sicherheitskriteriums angeboten werden, die er im einfachsten Fall nur bestätigen braucht. Hier können auch mehrere Parameter kombiniert werden (z.B. übereinstimmender Raum, Maximalabstand zwischen den Geräten, Kamera am Anwendungsgerät vorhanden, etc.).

Gemäß der Erfindung wird das Sicherheitskriterium spezifisch für jedes medizinische Gerät definiert. Damit kann vorteilhafterweise eine sehr hohe Flexibilität erzielt werden. Der technische Vorteil ist, dass medizinische Komplikationen und besondere Anwendungsszenarien sehr einfach und unkompliziert abgedeckt werden können (z.B. für einen Notfall-Dialysepatienten, der eine besonders intensive Betreuung benötigt, so dass die Fernbedienungsoptionen sehr eingeschränkt sein sollen).

Gemäß der Erfindung wird das Sichtkontaktsignal durch Auswertung eines RFID-Chips, eines NFC-Moduls, einer Kamera, durch Funktriangulation oder durch die Auswertung von digitalen Lageplänen ausgewertet. Bei den digitalen Lageplänen handelt es sich um Raum-Geräte-Zuordnungen in einem digitalen Format, das von einer Software eingelesen und verarbeitet werden kann. Mit Hilfe dieser Lagepläne, kann automatisch erfasst werden, welches Anwendungs- und medizinische Gerät sich in welchem Raum befindet bzw. welche weiteren Geräte sich ebenfalls in diesem Raum befinden. Diese Information kann beispielsweise verwendet werden, um aus der Menge der Anwendungsgeräte die Ziel-Anwendungsgeräte auszuwählen.

In einer weiteren, vorteilhaften Ausführungsform wird nämlich in einer Definitionsphase für jedes medizinische Gerät eine Auswahl von Ziel-Anwendungsgeräten konfiguriert, auf denen eine Fernbedienung bzw. -bearbeitung bei Erfüllung des Sicherheitskriteriums ausführbar ist. Dabei kann die Rolle zwischen Anwendungsgerät und medizinischem Gerät wechseln.

In einer weiteren, vorteilhaften Ausführungsform ist es vorgesehen, dass während der entfernten Bearbeitung von Meldungen auf dem Anwendungsgerät ein Fernbearbeitungszustand durch die Ausgabe eines Fernbearbeitungssignals ausgegeben wird, um die Fernbearbeitung nach außen zu signalisieren (z.B.: grafisch oder durch Popup eines Fenster mit der Kennzeichnung "Fernbearbeitung für das medizinische Gerät xyz" oder durch anderweitige Trennung der Benutzeroberfläche von dem Anwendungsgerät an sich und dem Anteil der Benutzeroberfläche, die für die Fernbearbeitung des medizinischen Gerätes reserviert ist).

In einer weiteren vorteilhaften Ausführungsform der Erfindung erfolgt das Überprüfen des Sicherheitskriteriums, indem zu dem Sichtkontaktsignal eine Sicherheitsstufe der zugeordneten Meldung ausgewertet wird. Damit umfasst das Überprüfen des Sicherheitskriteriums immer zusätzlich einen weiteren Verarbeitungsschritt, nämlich das Auswerten der jeweiligen Meldung und/oder eines Status der Meldung (z.B. niedriges, mittleres oder hohes Sicherheitsrisiko der Meldung). Ein unterschiedlicher Status einer Meldung erfordert die Anwendung von unterschiedlichen Sicherheitskriterien. In der vorstehend beschriebenen Ausführungsform der Erfindung umfasst das Verfahren somit folgenden Schritt:
- Auswerten (Bestimmen) einer Sicherheitsstufe der Meldung. Hier können vordefinierte Kategorien von Meldungen berücksichtigt werden. Dieser Schritt wird vorzugsweise nach Empfang der jeweiligen Meldung ausgeführt.

Gemäß einem weiteren Aspekt betrifft die Erfindung eine Fernsteuereinheit mit einer Identifikationseinrichtung nach Anspruch 6.

Die Identifikationseinrichtung kann dabei unter anderem eine optische Sensoranordnung, z.B. eine Kamera sein. Sie kann auch ein Identifikationsmodul zur Ausgabe von Signalen auf einer grafischen Benutzeroberfläche des Anwendungsgerätes sein. Das Identifikationsmodul kann softwarebasiert sein und den Datenaustausch zwischen Anwendungsgerät und fern zu steuerndem medizinischem Gerät nach einem vordefinierten Protokoll überwachen. So kann z.B. ein Aufforderungssignal (z.B. zur Ausführung einer bestimmten Eingabe) auf dem medizinischen Gerät generiert werden, das dann in Antwort darauf entsprechend auf dem Anwendungsgerät beantwortet werden muss. Dort wird dann von dem Identifikationsmodul überprüft, ob genau die erwartete Eingabe getätigt worden ist, sodass davon auszugehen ist, dass Sichtkontakt zwischen den Geräten besteht. Alternativ können zusätzliche Aufforderungsbefehle und -antworten gefordert werden. Alternativ oder kumulativ kann ein RFID-Modul oder ein NFC-Modul ausgebildet sein, um durch RFID- oder NFC-Kommunikation die Anwesenheit einer Person zu detektieren. Ein RFID/NFC-Empfänger ist dazu in oder an dem Anwendungsgerät ausgebildet und interagiert mit einem RFID/NFC-Sender, der von der Person getragen wird. Ansonsten kann die Identifikationseinrichtung eine akustische Sensoranordnung umfassen. So können auf dem medizinischen Gerät Befehle zur Eingabe eines akustischen Signals erzeugt und ausgegeben werden (direkt auf dem medizinischen Gerät), die dann durch entsprechende Eingaben auf dem Anwendungsgerät beantwortet werden. Alternativ kann die Anwesenheit über eine Funktriangulation oder über ein Kamerasystem detektiert werden.

In einer vorteilhaften Ausführungsform der Erfindung ist die Identifikationseinrichtung in einer der Fernsteuerphase zeitlich vorgelagerten Definitionsphase dazu ausgelegt, das Sicherheitskriterium zu definieren.

In einer weiteren, vorteilhaften Ausführungsform der Erfindung ist die Identifikationseinrichtung im Rahmen der Überprüfung des Sicherheitskriteriums dazu ausgebildet, zu dem Sichtkontaktsignal eine Sicherheitsstufe der jeweils zugeordneten Meldung auszuwerten. Damit ergibt sich der Vorteil, dass das Fernsteuerungsprozedur flexibler auf die jeweilige Sicherheitsstufe abgestellt und an diese angepasst werden kann. In einer weiteren, vorteilhaften Ausführungsform der Erfindung umfasst die Fernsteuereinheit eine Fernsteuersignaleinheit, die dazu ausgebildet ist, während der Ausführung der Bearbeitungsprozedur (zur entfernten Steuerung auf dem Anwendungsgerät) einen Fernbearbeitungszustand durch Ausgabe eines Fernbearbeitungssignals auf dem Anwendungsgerät zu kennzeichnen. Dies hat den Vorteil, dass die Fernbearbeitung oder Fernsteuerung des medizinischen Gerätes gerade auf dem jeweiligen Anwendungsgerät aktiviert ist und um somit den Fernsteuerungszustand nach außen zu repräsentieren und transparent zu machen, um Fehleingriffe zu vermeiden.

In einer weiteren, vorteilhaften Ausführungsform der Erfindung umfasst die Fernsteuereinheit eine Prüfeinheit, die dazu ausgebildet ist, zu bestimmen, für welche medizinischen Geräte das jeweilige Anwendungsgerät, dem die Fernsteuereinheit zugeordnet ist, zulässig aktivierbar ist. Die Prüfeinheit kann als Mikroprozessorbauteil und als Bestandteil der Identifikationseinrichtung ausgebildet sein. Die Prüfeinheit kann in Datenaustausch mit einer (z.B. zentralen) Datenbank stehen, in der die zulässigen Zuordnungen zwischen dem jeweils fernzusteuernden medizinischen Gerät und dem Anwendungsgerät hinterlegt sin. Dies hat den technischen Effekt, dass die zulässigen Zuordnungen zentral administriert und einheitlich für alle Geräte angewendet werden können.

Die Prüfeinheit kann zum einen - in der Definitionsphase - zum Definieren von zulässigen Zuordnungen von medizinischem Gerät und Anwendungsgerät/Fernsteuergerät dienen. Die Prüfeinheit kann zum anderen - in einer Steuerphase - zum Überprüfen angewendet werden, ob das jeweilige Anwendungsgerät auch zur Fernsteuerung des medizinischen Gerätes eingesetzt werden kann.

Gemäß einem weiteren Aspekt betrifft die Erfindung ein medizinisches Anwendungsgerät mit einer Fernsteuereinheit, wie vorstehend beschrieben. Dabei kann das Anwendungsgerät auch als mobiles Endgerät ausgebildet sein.

Ein wesentlicher Gedanke der Erfindung ist darin zu sehen, dass durch das Bereitstellen einer gesicherten Fernbearbeitungsfunktionalität von Meldungen des fern zu bedienenden medizinischen Gerätes auf einem entfernten Anwendungsgerät die Gefahr von Kontaminationen oder Kreuzkontaminationen verringert werden kann. Des Weiteren kann die erforderliche Ruhe in einem Dialysezentrum mit mehreren Behandlungsplätzen beibehalten werden, auch wenn nur wenig Personal zur Verfügung steht, indem akustische Alarmsignale vermieden werden können, da die jeweilige Meldung auch auf einem anderen Gerät aus bedient werden kann. Damit kann vorteilhafterweise auch die Reaktionszeit verkürzt werden, da ein ansonsten notwendiger Desinfektionsprozess beim Gerätewechsel vermieden werden

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

- Fig. 1: zeigt in einer Schemazeichnung eine Dialysestation mit mehreren Dialysegeräten und einem oder mehreren Anwendungsgeräten.
- Fig. 2: beschreibt schematisch den Signalaustausch zwischen einem Anwendungsgerät und einem medizinischen Gerät zur Dialyse.
- Fig. 3: ist ein Ausführungsbeispiel, bei dem ein Anwendungsgerät mehrere medizinische Geräte steuert und
- Fig. 4: ist ein Ausführungsbeispiel, bei dem mehrere Anwendungsgeräte ein medizinisches Gerät steuern.

### Detaillierte Beschreibung der Erfindung

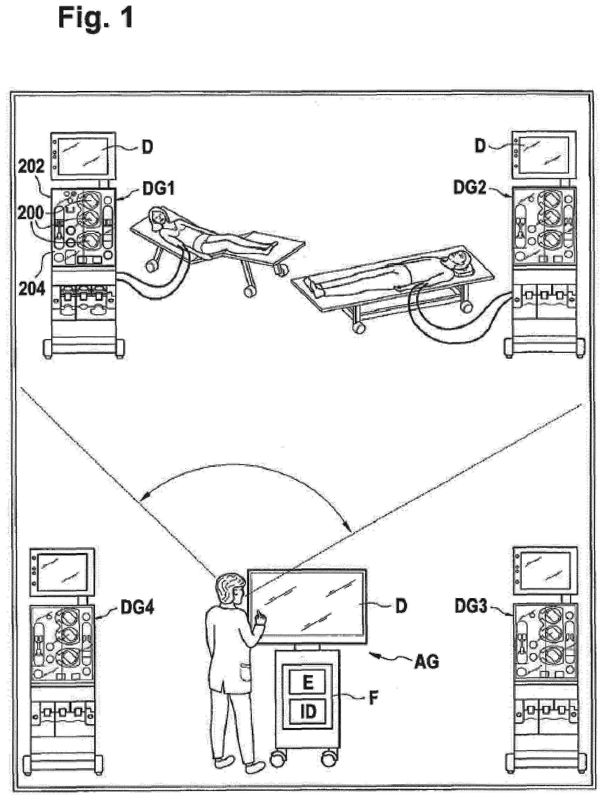

**Figur 1** zeigt eine typische Dialysestation mit mehreren medizinischen Geräten DG, AG insbesondere Dialysegeräten und zugeordneten Patientenliegen mit Patienten. In der Dialysestation können noch weitere Geräte betrieben werden, an denen Anwender arbeiten und die im Folgenden als Anwendungsgeräte AG bezeichnet werden. Ein Dialysegerät DG kann auch die Rolle eines Anwendungsgerätes AG haben, falls ein Anwender an ihm arbeitet und es bedient. Ebenso kann auch ein Anwendungsgerät AG die Rolle eines Dialysegerätes DG haben, wenn z.B. der Arzt gerade den Patienten an diesem Dialysegerät DG behandelt und dasselbe dazu bedient. Ein Anwendungsgerät AG muss somit nicht zwangsläufig ein Dialysegerät sein, sondern kann z.B. auch ein Zentralcomputer, ein lokales elektronisches Steuergerät oder ein mobiles Gerät, wie ein Laptop oder ein Smartphone sein.

Ein Dialysegerät DG ist im Stand der Technik bekannt. Hierzu wird auf die DE 10 2011 107 795 verwiesen, in der unterschiedliche Blutbehandlungsgeräte beschrieben sind. Die DE 197 426 33 der Anmelderin zeigt weiterhin ein Beispiel für ein Dialysegerät, das als Ein- und Ausgabeschnittstelle einen Touchscreen umfasst.

Figur 1 zeigt in einer Prinzip-Darstellung ein Dialysegerät als Vertreter eines medizinischen Gerätes DG. Das Dialysegerät wird über ein angeschlossenes oder integriertes Display D bedient und gesteuert. Das Display D dient als Ein- und Ausgabeschnittstelle bzw. als Benutzerschnittstelle. Üblicherweise ist an das medizinische Gerät DG eine Steuerungseinheit über eine geeignete Datenverbindung angeschlossen. Das Dialysegerät umfasst, wie in Figur 1 schematisch gezeigt, als zentrales Element ein extrakorporales Behandlungsmodul 202. Dieses kann mit einer transparenten Abdeckung 204, hier in Form einer zweiflügeligen Türe vor ungewollten Berührungen gesichert sein. Das extrakorporale Behandlungsmodul 202 umfasst eine Vielzahl von Betriebsmitteln 200 in Form von unterschiedlichen Pumpen (z.B. Blut, Substituat), Ventilen, Spritzen, Halterungen, Aufnahmen und dergleichen. Die Betriebsmittel 200 können sich an unterschiedlichen Positionen entweder innerhalb des medizinischen Gerätes DG oder außerhalb (an einer angeschlossenen Baueinheit) befinden, also auch außerhalb des extrakorporalen Behandlungsmoduls 202. Darüber hinaus können die Betriebsmittel 200 selbst auch als externe, separate Vorrichtung über eine entsprechende Verbindung und eine Datenverbindung an das Dialysegerät angeschlossen sein. In dem in Figur 1 gezeigten Ausführungsbeispiel ist im oberen mittigen Bereich des extrakorporalen Behandlungsmoduls 202 eine Blutpumpe dargestellt. Darunter befindet sich eine Substituatpumpe und auf der linken Seite unter einer Heparinpumpe ein Substituatport. Am unteren Teil des extrakorporalen Behandlungsmoduls 202 befinden sich weitere Betriebsmittel 200. Des Weiteren können weitere Messeinheiten, Anschlüsse und/oder weitere Bauteile in dem extrakorporalen Behandlungsmodul 202 ausgebildet sein.

Das Anwendungsgerät AG umfasst eine Fernsteuereinheit F mit einer Identifikationseinrichtung ID und einer Eingangsschnittstelle E. Die Fernsteuereinheit F dient zur entfernten Bearbeitung von Meldungen S2 und damit zum Fernsteuern des medizinischen Gerätes DG auf dem entfernten Anwendungsgerät AG. Dies betrifft insbesondere das Bearbeiten von Meldungen S2, die den Betrieb des fern zu bedienenden medizinischen Gerätes DG betreffen. Die Eingangsschnittstelle E dient zum Erfassen eines Aktivierungssignals S2 und zum Empfangen der jeweiligen Meldung S2 des medizinischen Gerätes DG. Die Identifikationseinrichtung ID ist dazu bestimmt, ein Sicherheitskriterium zu überprüfen und dabei automatisch ein Sichtkontaktsignal auszuwerten. Bei positiven Ergebnis dieser Überprüfung des Sicherheitskriteriums ist die Fernsteuereinheit F für einen vorkonfigurierbaren Zeitraum zur entfernten Bearbeitung der jeweiligen Meldung S2 bestimmt, solange das Sicherheitskriterium erfüllt ist.

Im Dialysebetrieb wird aus Sicherheitsgründen der Status des Dialysegerätes DG oder seiner Betriebsmittel 200 erfasst. Entsprechende Statusmeldungen oder Meldungen allgemeine Art werden dazu auf dem Display D des jeweiligen Dialysegerätes DG ausgegeben. Hier werden unterschiedliche Sicherheitsstufen von Meldungen unterschieden. In einem Ausführungsbespiel kann zwischen 3 Klassen von Meldungen differenziert werden:
1. Alarmmeldungen mit hoher Priorität, die ein Sicherheitsrisiko bergen und die unmittelbar beantwortet werden müssen.
2. Alarmmeldungen mit geringer Priorität und
3. einfache Meldungen, die nicht notwendigerweise innerhalb einer bestimmten Zeitphase beantwortet werden müssen.

Die Definition der Sicherheitsstufen ist erfindungsgemäß konfigurierbar, so dass in anderen Beispielen mehr oder weniger Sicherheitsstufen erfasst und zur Überprüfung eines Sicherheitskriteriums verrechnet werden. In der Definitionsphase können z.B. unterschiedliche Festlegungen bezüglich der Sicherheitsstufen getroffen werden: Meldungen der Klasse 1 erfordern eine direkte Quittierung mit einer Anwenderanwesenheit vor Ort, also am meldenden Dialysegerät DG selbst (keine Option für eine Fernbearbeitung der Meldung), während Meldungen der Klasse 2 eine indirekte Quittierung erlauben, allerdings nur unter einem Sicherheitskriterium, das festlegt, dass sich das Anwendungsgerät AG im selben Raum befinden muss. Meldungen der Klasse 3 können auch eine indirekte Quittierung erlauben, ohne dass das Anwendungsgerät bestimmte Positionsauflagen erfüllen muss. Üblicherweise werden die Meldungen auf dem jeweiligen Dialysegerät DG ausgegeben, auf dem sie erfasst werden, insbesondere auf dessen Monitor D. Erfindungsgemäß wird es möglich, für den Arzt oder den Anwender eine weitere Flexibilität bereitzustellen. Die Meldung wird erfindungsgemäß unter bestimmten Umständen (Sicherheitskriterium auf Ziel-Anwendungsgerät erfüllt) auf einem Anwendungsgerät AG ausgegeben, an dem der Anwender gerade arbeitet. Um Sicherheitsanforderungen zu genügen, wird die Meldung jedoch nicht auf einem beliebigen Anwendungsgerät AG ausgegeben, sondern nur auf dezidierten Geräten. Dafür kann z.B. ein einer Definitionsphase im Vorfeld für jedes Dialysegerät DG eine Menge von möglichen Ziel-Anwendungsgeräte bestimmt werden, die prinzipiell zur Fern-Bearbeitung von Meldungen von und für das jeweilige Dialysegerät DG in Frage kommen. Hier kann die Organisationseinheit (z.B. das Krankenhaus) Regelungen spezifizieren und z.B. festlegen, dass grundsätzlich nur die Ziel-Anwendungsgeräte in Frage kommen, die sich im selben Raum wie das jeweilige Dialysegerät DG befinden, so dass prinzipiell ein Sichtkontakt zwischen Anwender, der an dem Anwendungsgerät AG arbeitet, und dem jeweiligen Dialysegerät DG herstellbar ist.

Als Sicherheitskriterium wird in der Erfindung festgelegt, dass eine Identifikationseinrichtung ID, insbesondere eine Personenidentifikationseinrichtung an oder auf dem Anwendungsgerät AG bereitgestellt wird, die dazu bestimmt ist, zu identifizieren, ob sich ein Anwender vor dem Anwendungsgerät AG befindet. Bei der Identifikationseinrichtung ID kann es sich z.B. um einen optischen Sensor oder um eine Kamera handeln. In einer anderen Ausführungsform der Erfindung kann mittels eines Identifikationsmoduls auch ein Signalaustausch zwischen Anwendungsgerät AG und dem fern zu bedienendem Dialysegerät DG initiiert und überprüft werden, um sicherzustellen, dass der Anwender vor dem Anwendungsgerät AG auch die Befehle oder Aufforderungen des Dialysegerätes DG beantworten kann. So kann beispielsweise ein Signalaustausch nach dem Challenge-Response Verfahren implementiert werden, bei dem das fern zu bedienendem Dialysegerät DG eine Aufforderung an das jeweilige Anwendungsgerät AG sendet und dieses dann daraufhin seitens des Anwenders veranlasst wird, mit einem Antwortsignal an das Dialysegerät DG zu antworten. Ebenso kann ein Signalaustausch zwischen unterschiedlichen Gerätebauteilen aufgesetzt werden, indem die Aufforderung seitens des Dialysegerätes DG z.B. eine Aufforderung für eine Geste oder Bewegung ist, die von der Kamera des Anwendungsgerätes AG erfasst wird. Bei korrekter Beantwortung gilt das Sicherheitskriterium als erfüllt, so dass eine Fernbedienung auf dem Anwendungsgerät AG ausgeführt werden kann.

Um den Aspekt des Sicherheitskriteriums des Sichtfeldes auch in der Figur 1 zu repräsentieren, ist ausgehend von dem Anwendungsgerät AG ein Sichtkegel eingezeichnet, der in diesem Beispiel die Dialysegeräte DG1 und DG2 umfasst, während DG3 und DG4 sich außerhalb des Sichtfeldes befinden. Laut Konfiguration in der Definitionsphase kommt zwar Anwendungsgerät AG grundsätzlich als Ziel-Anwendungsgerät zur Fernbedienung von dem Dialysegerät DG3 im Frage, aber es befindet sich außerhalb des Sichtfeldbereiches und kann von daher das Sicherheitskriterium nicht erfüllen. Das Dialysegerät DG4 befindet sich beispielsweise in einer abgelegenen Position, so dass es bereits im Vorfeld nicht als Ziel-Anwendungsgerät in Frage kommt.

Das Verfahren untergliedert sich in eine Definitionsphase, in der grundlegende Definitionen und Einstellungen konfiguriert werden und in eine Steuerphase. Die Definitionen können durch den Anwender erfolgen. Zum Beispiel kann konfiguriert werden, welche Regeln für die Bestimmung der Ziel-Anwendungsgeräte AG für jeweils ein Dialysegerät DG gelten sollen. Des Weiteren werden in der Definitionsphase die Parameter für das Sicherheitskriterium konfiguriert. Es können auch unterschiedliche Parameter mit Bedingungen verknüpft werden (z.B. Sicherheitskriterium = Parameter 1 UND Parameter 2 ODER Parameter 3 UND NICHT Parameter 4). Dazu kann ein Menü auf der Benutzeroberfläche bereitgestellt werden, in dem eine oder mehrere Option(en) auswählbar sind, wie z.B. "Position des Anwendungsgerätes im selben Raum", "Kamera auf dem Anwendungsgerät vorhanden und einsatzfähig", Mindestabstand zwischen den Geräten nicht über x Meter" etc. In der zeitlich nachgelagerten Steuerphase wird dann ein konkretes Dialysegerät DG fernbedient bzw. ferngesteuert, indem alle oder ausgewählte Befehle oder Eingaben nicht mehr auf dem Display D des Dialysegerätes DG erfasst werden, sondern auf einem bestimmten Anwendungsgerät AG, welches das vordefinierte Sicherheitskriterium erfüllt und solange es selbiges erfüllt.

In der Definitionsphase kann z.B. auch konfiguriert werden, dass Meldungen wahlweise immer noch unmittelbar auf dem jeweiligen Dialysegerät DG quittiert oder beantwortet werden können, wenn sich der Anwender z.B. gerade zufällig zum Zeitpunkt der Meldung vor dem jeweiligen Meldungsauslösenden Dialysegerät DG befindet und optional und damit sozusagen zusätzlich eine weitere Fernbedienung auf dem Anwendungsgerät AG möglich wird. Ebenso kann konfiguriert werden, dass im Fall einer Fernbedienung dann nur eine Bedienung durch das Anwendungsgerät AG akzeptiert wird und keine unmittelbare Bedienung auf dem Dialysegerät DG. Weiterhin kann auch ein Zeitintervall konfiguriert werden, das festlegt, in welchem Zeitraum bestimmte Meldungen beantwortet und quittiert werden müssen.

In der Definitionsphase ist es auch konfigurierbar, dass zwischen unterschiedlichen Sicherheitsstufen differenziert wird. So wird eine Meldung mit hoher Priorität (z.B. Alarmsignal) anders bearbeitet als eine Meldung mit niedriger Priorität. Eine Meldung mit hoher Priorität kann z.B. zusätzlich noch mit dem Auslösen eines optischen (oder alternativen) Alarmsignals auf dem Dialysegerät DG oder auf dem Anwendungsgerät AG gekoppelt sein. Dies hat den technischen Effekt, dass der Anwender im Fall eines relevanten Alarms über mehrere, unterschiedliche Signaltypen informiert wird.

Die Fernbedienung umfasst in der Regel das Quittieren von Meldungen, die auf dem Display D ausgegeben werden. Dazu weist auch das Anwendungsgerät AG ein Display D auf, auf dem die Meldungen ausgegeben werden und auf dem das Bearbeiten der ausgegebenen Meldung erfolgt, insbesondere über die Eingabe eines Quittierungssignals. Die Fernbedienung kann aber auch mehrere Schritte umfassen, die eine Abfolge von Eingaben auf dem Touchscreen erfordern.

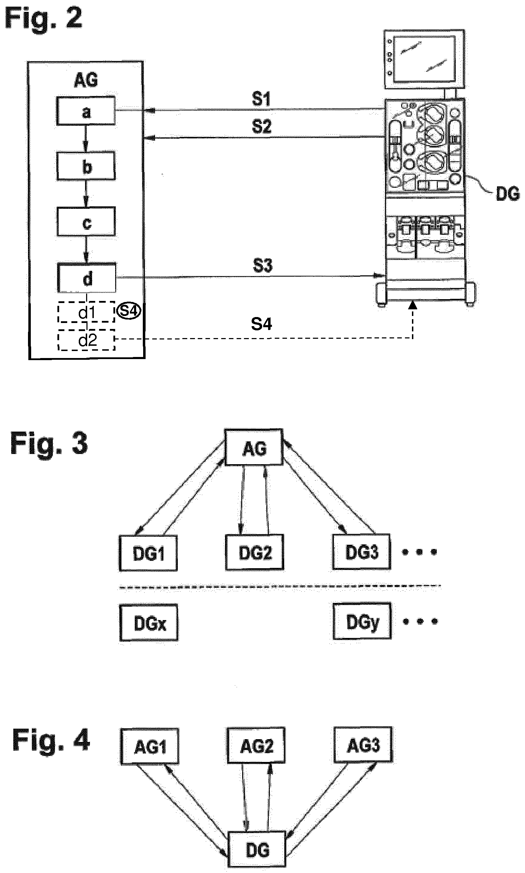

Das erfindungsgemäß Verfahren wird im Folgenden unter Bezugnahme auf **Figur 2** näher erläutert. Nach Abschluss der Definitionsphase sind alle Konfigurationen bestimmt. In der Steuerphase soll das Dialysegerät DG über das (bzw. auf dem) Anwendungsgerät AG ferngesteuert werden, weil bspw. der Arzt gerade dort arbeitet und bei Meldungen auf dem Dialysegerät DG nicht die Gefahr von Kreuzkontaminationen hervorrufen möchte durch Kontakt mit dem "fremden" Display D des Dialysegerätes DG mit Erregern ohne vorherige Desinfektion. Diese Gefahr kann ausgeschlossen werden, indem der Arzt seinen Aktionsbereich auf dem Anwendungsgerät AG nicht "verlässt" und dennoch eine Steuerung des Dialysegerätes DG ausführen kann. Dazu wird zunächst ein Aktivierungssignal S1 auf dem Dialysegerät DG generiert und an das Anwendungsgerät AG übertragen und dort in **Schritt a** empfangen. Dies hat den technischen Vorteil, dass nur eine wirklich intendierte Fernbedienung ausgeführt wird und Fehler durch ungewollte Fremdbedienungen vermieden werden können. So wird z.B. eine Fernbedienung ausgeschlossen, wenn kein Aktivierungssignal S1 versendet worden ist. Damit kann die Sicherheit erhöht werden. Erst nach Erhalt des Aktivierungssignals S1 kann eine Fernbedienung ausgelöst werden. Dazu wird eine konkrete Meldung S2 auf dem Dialysegerät DG generiert und an das Anwendungsgerät AG gesendet. In **Schritt b** kann die Meldung S2 auf dem Anwendungsgerät AG empfangen werden und auf dem Display D des Anwendungsgerätes AG ausgegeben werden. Vorzugsweise ist ein drahtloser Nachrichtenaustausch zwischen Dialysegerätes DG und Anwendungsgerät AG vorgesehen (z.B. Mobilfunkverbindung, Bluetooth etc.) oder ein drahtgebundener Nachrichtenaustausch (z.B. über eine Netzwerkverbindung).

Optional kann die Meldung S2 auch direkt auf dem Display D des Dialysegerät DG dargestellt werden. In **Schritt c** wird ein (vordefiniertes) Sicherheitskriterium für die Meldung S2 und für das jeweilige Dialysegerät DG überprüft. Insbesondere wird überprüft, ob ein Sichtkontakt zwischen dem an dem Anwendungsgerät AG arbeitenden Anwender und dem fern zu bedienenden Dialysegerät DG besteht. Zusätzlich kann optional die Sicherheitsstufe der jeweiligen Meldung S2 geprüft werden, wenn z.B. in der Definitionsphase bestimmt ist, dass Meldungen mit hoher Priorität nur direkt auf dem Dialysegerät DG selbst quittiert werden können und grundsätzlich einer Fernquittierung nicht zugänglich sind, während Meldungen mit niedriger Priorität einer Fernquittierung zugänglich sind. In komplexeren Ausführungsformen der Erfindung können hier noch weitere Bedingungen und Verknüpfungen definiert werden. Nachdem das Sicherheitskriterium auf Einhaltung überprüft worden ist, erfolgt in **Schritt d** das Ermöglichen des Bearbeitens der Meldung S2 auf dem Anwendungsgerät AG mit der Erfassung des Quittierungssignals S3 auf dem Anwendungsgerät AG und dessen Versendung an das Dialysegerät DG. Insbesondere können zwei Schritte d1 und d2 ausgeführt werden, die in Figur 2 als Strichlinie dargestellt sind. In Schritt d1 wird das Quittierungssignal S4 auf dem Anwendungsgerät AG erfasst, vorzugsweise auf dem Touchscreendisplay D. In Schritt d2 wird das erfasste Quittierungssignal S4 an das Dialvseaerät DG zur Steueruna übertragen und dort entsprechend verarbeitet. Wesentlich ist, dass die Quittierung (der Meldung S2) auf dem Anwendungsgerät AG nach erfolgreicher Überprüfung des Sicherheitskriteriums erfolgt. Nach der Quittierung wird das Quittierungssignal S3, S4 an das Dialysegerät DG zur Steuerung übertragen. Bei den Quittierungssignalen S3 und S4 handelt es sich um dasselbe Signal, das zur Verdeutlichung in Fig. 2 nur ein anderes Bezugszeichen trägt.

**Figur 3** zeigt ein Ausführungsbeispiel, in dem ein bestimmtes Anwendungsgerät AG grundsätzlich mehrere unterschiedliche Dialysegeräte DG1, DG2, DG3,...DGx, DGy steuern kann. Wie in Figur 3 durch die horizontal verlaufende gepunktete Linie dargestellt, erfüllt dabei allerdings das Anwendungsgerät AG nur für einen Teil der Dialysegeräte DG1, DG2, DG3 das Sicherheitskriterium (z.B. Sichtkontakt). Die Dialysegeräte DGx und DGy befinden sich außerhalb des Sichtfeldes und können daher von dem Anwendungsgerät AG nicht gesteuert werden. In der Definitionsphase kann konfiguriert werden, ob die Fernbearbeitung (Fernquittierung) von Meldungen von den das Sicherheitskriterium erfüllenden Dialysegeräten DG in derselben Zeitphase erfolgen darf, sozusagen in einem Interleaved Modus oder nur sequentiell. Im ersten Fall wird durch zusätzliche Maßnahmen sichergestellt, dass die jeweilige Fernsteuerung auf dem Anwendungsgerät AG auch dem richtigen Dialysegerät DG zugeordnet ist. Dies kann vorzugsweise durch einen zusätzlichen Signal- oder Datenaustausch gemäß einem vordefinierten Protokoll zwischen Anwendungsgerät AG und Dialysegerät DG implementiert werden.

**Figur 4** zeigt ein Ausführungsbeispiel, in dem ein Dialysegerät DG von unterschiedlichen Anwendungsgeräten AG1, AG2, AG3 ferngesteuert werden kann, wenn sie das Sicherheitskriterium erfüllen. Auch hier kann konfiguriert sein, dass die Fernsteuerung sozusagen im Parallelmodus auf den unterschiedlichen Geräten AG1, AG2, AG3 möglich ist, um der Schwester oder dem Arzt mehr Flexibilität zu geben. Er kann dann dasjenige Anwendungsgerät AG zur Bedienung bestimmen, das für ihn am nächsten oder am besten geeignet ist. Dazu muss aber sichergestellt werden, dass das Aktivierungssignal S1 und die jeweiligen Meldungen S2 an alle Anwendungsgeräte AG1, AG2, AG3 übertragen werden.

Die beiden in wechselnder Richtung weisenden Pfeile zwischen jeweils den/dem Anwendungsgerät(en) AG1, AG2, AG3 und den/dem Dialysegerät(en) DG1, DG2, DG3 in den Figuren 3 und 4 sollen jeweils die ausgetauschten Nachrichten repräsentieren, insbesondere die Meldung S1 und das Bearbeitungs- oder Quittierungssignal S3.

In einer bevorzugten Ausführung wird die Fernsteuerungsfunktionalität bestimmt. Dies kann hinsichtlich Umfang (welcher Modus von Meldungen kann einer Fernbearbeitung zugänglich sein) oder hinsichtlich einer Zeitphase erfolgen (in welchem Zeitraum ist die Fernbearbeitung zulässig).

Zur Vermeidung von Kreuzkontamination und zur Erhöhung des Bedienungskomforts wird in einer Ausführungsform der Erfindung eine Möglichkeit geschaffen, den kompletten Bedienbildschirm einer Dialysemaschine auf den Bildschirm eines Anwendungsgerätes zu bringen, wobei das Anwendungsgerät sich entfernt von der Dialysemaschine befindet. Alternativ können nur Bestandteile des Bildschirms auf das Anwendungsgerät AG übertragen werden, die z.B. für die Meldung S2 relevant sind. Es wird dabei automatisch geprüft, ob das Anwendungsgerät AG auch über die notwendigen Funktionalitäten zur Darstellung des Bedienbildschirms und zum Erfassen von entsprechenden Eingaben hat und ob es das Sicherheitskriterium erfüllt. Eine direkte Bearbeitung von Meldungen S2 auf dem meldenden Dialysegerät DG ist somit nicht mehr zwingend erforderlich. Während der Arbeit mit einem Kontakt zu einem Patienten an einem Anwendungsgerät AG ist erfindungsgemäß eine sichere, durch feste Regeln festgelegte Fern-Bedienung an genau diesem Anwendungsgerät AG für ein entferntes, meldendes Dialysegerät DG möglich, ohne die Gefahr von Kreuzkontamination hervorzurufen.

Hierzu sind die Geräte bzw. Maschinen oder elektronischen Apparate DG, AG miteinander vernetzt (z.B. über ein lokales Netzwerk oder ein WAN oder über das Internet etc.). Jede Maschine erkennt durch die ihm zugeordnete Identifikationseinrichtung (z.B. durch berührungslose Techniken wie NFC oder RFID), ob ein Anwender so in der Nähe ist, dass er das entfernte Dialysegerät DG einsehen und fern-bedienen kann. Zugleich wird die Berechtigung des sich in der Nähe befindlichen Anwenders berücksichtigt: In einer z.B. durch NFC, RFID oder durch Einlesen einer SmardCard übermittelten Kennung ist das Berechtigungsniveau des Anwenders codiert bzw. wird auf Basis der übermittelten Kennung aus einer Datenbank abgerufen. So können Eingaben von unterschiedlichen Personengruppen unterschiedlich verarbeitet werden. Einer Eingabe eines Arztes kann z.B. eine höhere Priorität zugeordnet werden als derjenigen einer Schwester oder umgekehrt.

Im Falle von Meldungen und Alarmen an Dialysegeräten DG kann z.B. nur an solchen Anwendungsgeräten AG eine Meldung mit der Position des meldenden Dialysegerätes DG ausgegeben werden, bei denen ein Anwender in der Nähe erfasst worden ist (z.B. mit der Identifikationseinrichtung ID). Die Position der meldenden Maschine kann z.B. über das Auswerten eines Raumplans erfolgen. Das gleiche Verfahren kann bei anderen, ortsfesten Anwendungsgeräten AG verwendet werden. Bei mobilen Anwendungsgeräten AG kann die aktuelle Position des Anwenders automatisch durch entsprechende Sensoren erfasst werden. Dieses kann z.B. durch Ortung mittels der in den Dialysegeraten befindlichen Sensorik oder durch Funktriangulation erfolgen.

Des Weiteren ist für die Ausgabe von Meldungen und Alarmen für jede Meldung S2 definiert, in welche Umgebung sie für einen Remote-Zugriff übertragen werden dürfen. So kann z.B. festgelegt sein, dass ein kritischer Alarm wie bspw. eine detektierte Nadeldislokation des venösen Zugangs eines Dialysepatienten nur an Anwendungsgeräte AG im unmittelbaren Umkreis des meldenden Dialysegerätes DG übermittelt werden darf, während z.B. Warnungen für das Nichterreichen des Behandlungsziels bzgl. einer Dialysequalität auch über größere Entfernungen übertragen werden dürfen. Damit wird vorteilhafterweise für jede Bedien-/Alarmsituation eine Umgebung definiert, die eine der unmittelbaren Bedienung am Dialysegerät DG gleichwertige Bedienung über einen Remote-Zugriff ermöglicht.

Um sicher zu vermeiden, dass der Anwender Remotezugriffe auf Maschinen ausführt, die er nicht im Sichtbereich bzw. Blickfeld hat (und dann den Zustand des Patienten nicht beurteilen könnte), wird für jedes Dialysegerät DG eine Umgebung mit den für den Remotezugriff zulässigen Ziel-Anwendungsgeräten AG definiert. Im einfachsten Fall ist dies ein Behandlungsraum. Diese Definition kann in einer zentralen Datenbank hinterlegt werden und damit für alle Geräte AG, DG einheitlich zur Anwendung kommen.

In einem Ausführungsbeispiel ist das Identifikationsmodul der Fernsteuereinheit F dazu ausgebildet, einen Raumplan auf dem Anwendungsgerät AG anzuzeigen. Dies kann alternativ zu dem Aktivierungssignal S1 erfolgen und kennzeichnet dann, dass ein Remote-Eingriff für ein entferntes Dialysegerät DG initiiert werden soll. Auf dem eingeblendeten Raumplan drückt der Anwender auf den Platz des betreffenden meldenden Dialysegerätes DG. Das Identifikationsmodul veranlasst das Dialysegerät DG daraufhin, bspw. ein optisches Signal, wie eine Ampeldarstellung (z.B. Blinksequenz) ein Zeichen zur Identifizierung zu senden. Daraufhin wird der Bildschirm des Dialysegerätes DG auf den Bildschirm des Anwendungsgerätes AG, vor der der Anwender steht, übertragen.

Durch das Fernbearbeitungssignal (z.B. in Form eines speziellen Rahmens um den Anwenderbildschirm) wird dem Anwender signalisiert dass es sich um einen Remotezugriff handelt. Nun kann der Anwender von seiner aktuellen Position an dem Anwendungsgerät AG aus das andere Dialysegerät DG bedienen. Nach Ende des Remotezugriffs zeigt die "fernbearbeitete" Maschine an, dass sie wieder "frei" ist.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für Dialysegeräte angewendet werden kann, sondern auch für andere medizinische Geräte, die eine Benutzeroberfläche zum Betrieb des Gerätes aufweisen. Des Weiteren können die Bauteile der Fernsteuereinheit F auch auf mehreren physikalischen Produkte verteilt realisiert sein.

### BEZUGSZEICHEN

- DG: Dialysegerät
- 200: Betriebsmittel
- 204: transparente Abdeckung
- 202: extrakorporales Blutbehandlungsmodul

- AG: Anwendungsgerät
- D: Display
- F: Fernsteuereinheit
- ID: Identifikationseinrichtung
- E: Eingangsschnittstelle
- S1: Aktivierungssignal
- S2: Meldung
- S3, S4: Bearbeitungssignal, insbesondere Quittierungssignal

- a: Erfasse Aktivierungssignal
- b: Empfange Meldung
- c: Überprüfe Sicherheitskriterium
- d: entferntes Bearbeiten

## Patentansprüche

1. Verfahren zum Steuern eines medizinischen Gerätes (DG) durch das Bearbeiten von Meldungen (S2) auf einer Fernsteuereinheit (F) eines Anwendungsgerätes (AG), wobei sich das Anwendungsgerät (AG) entfernt, aber in Sichtkontakt von dem medizinischen Gerät (DG) befindet und wobei die Meldungen den Betrieb des medizinischen Gerätes (DG) betreffen, mit folgenden Verfahrensschritten:
- Konfiguration eines Sicherheitskriteriums, wobei das Sicherheitskriterium spezifisch für jedes medizinische Gerät (DG) definiert ist,
- Erfassen der Anwesenheit eines Anwenders vor dem Anwendungsgerät (AG)
mittels einer Identifikationsreinrichtung (ID), die an oder auf dem Anwendungsgerät (AG) bereitgestellt ist und;
- Erfassen (a) eines Aktivierungssignals auf dem Anwendungsgerät (AG);
- Empfangen (b) der jeweiligen Meldung (S2) des medizinischen Gerätes (DG) auf dem Anwendungsgerät (AG);
- Mittels der Identifikationsreinrichtung (ID): Überprüfen (c) des Sicherheitskriteriums mit einer Auswertung eines Sichtkontaktsignals, welches einen Zustand repräsentiert, ob Sichtkontakt zwischen dem Anwendungsgerät (AG), an dem der ihm zugeordnete Anwender arbeitet, und dem medizinischen Gerät (DG) und/oder einem am medizinischen Gerät (DG) angeschlossenen Patienten besteht, wobei das Sichtkontaktsignal durch Auswertung eines RFID-Chips, eines NFC-Moduls, einer Kamera, durch Funktriangulation oder durch die Auswertung von digitalen Lageplänen ausgewertet wird und bei positivem Überprüfungsergebnis:
∘ Bereitstellen einer Bearbeitungsprozedur zum entfernten Bearbeiten (d) der jeweiligen Meldung (S2) auf dem Anwendungsgerät (AG), solange das Sicherheitskriterium erfüllt ist.

2. Verfahren nach Patentanspruch 1, bei dem das Sicherheitskriterium in einer Definitionsphase konfiguriert wird.

3. Verfahren nach einem der vorhergehenden Patentansprüche, bei dem das Überprüfen des Sicherheitskriteriums erfolgt, indem zusätzlich zu dem Sichtkontaktsignal eine Sicherheitsstufe der zugeordneten Meldung (S2) ausgewertet wird.

4. Verfahren nach einem der vorhergehenden Patentansprüche, bei dem in einer Definitionsphase für jedes medizinische Gerät (DG) eine Auswahl von Ziel-Anwendungsgeräten konfiguriert wird, auf denen jeweils eine Bearbeitungsprozedur zum Fernbearbeiten bereitgestellt wird, welche bei Erfüllung des Sicherheitskriteriums ausführbar ist.

5. Verfahren nach einem der vorhergehenden Patentansprüche, bei dem während der Ausführung der Bearbeitungsprozedur ein Fernbearbeitungszustand durch die Ausgabe eines Fernbearbeitungssignals auf dem Anwendungsgerät (AG) gekennzeichnet wird.

6. Fernsteuereinheit (F) für ein medizinisches Anwendungsgerät (AG) zum Fernsteuern eines medizinischen Gerätes (DG), wobei sich das Anwendungsgerät (AG) entfernt, aber in Sichtkontakt von dem medizinischen Gerät (DG) befindet, durch Bearbeiten von Meldungen (S2), die den Betrieb des medizinischen Gerätes (DG) betreffen, mit:
- einer Eingangsschnittstelle (E), die zum Erfassen eines Aktivierungssignals (S2) bestimmt ist und die weiterhin zum Empfangen der jeweiligen Meldung (S2) des medizinischen Gerätes (DG) bestimmt ist, und mit
- einer Identifikationseinrichtung (ID), die an oder auf dem Anwendungsgerät (AG) bereitgestellt ist und, die dazu bestimmt ist,
∘ die Anwesenheit eines Anwenders vor dem Anwendungsgerät (AG) zu erfassen und
∘ ein Sicherheitskriterium spezifisch für jedes medizinische Gerät (DG) zu definieren und
∘ das Sicherheitskriterium zu überprüfen und dabei automatisch ein
Sichtkontaktsignal auszuwerten, wobei das Sichtkontaktsignal einen Zustand repräsentiert, ob Sichtkontakt zwischen dem Anwendungsgerät (AG), an dem der ihm zugeordnete Anwender arbeitet, und dem medizinischen Gerät (DG) und/oder einem am medizinischen Gerät (DG) angeschlossenen Patienten besteht; und
∘ wobei die Identifikationseinrichtung (ID) zur Auswertung des Sichtkontaktsignals einen RFID-Chip, ein NFC-Modul, eine Kamera und/oder digitale Lagepläne auswertet und/oder ein Prozessorbauteil umfasst, das zur Ausführung einer Funktriangulation bestimmt ist;
- wobei die Fernsteuereinheit (F) bei positivem Überprüfungsergebnis zum Ausführen eine Bearbeitungsprozedur zur entfernten Bearbeitung der jeweiligen Meldung (S2) bestimmt ist, solange das Sicherheitskriterium erfüllt ist.

7. Fernsteuereinheit (F) nach einem der vorangehenden auf die Fernsteuereinheit (F) gerichteten Patentansprüche, bei der die Identifikationseinrichtung (ID) in einer Definitionsphase dazu ausgelegt ist, das Sicherheitskriterium zu definieren oder das Sicherheitskriterium aus einer angeschlossenen Datenbank einzulesen.

8. Fernsteuereinheit (F) nach einem der vorangehenden auf die Fernsteuereinheit (F) gerichteten Patentansprüche, bei der die Identifikationseinrichtung (ID) im Rahmen der Überprüfung des Sicherheitskriteriums dazu ausgebildet ist, zu dem Sichtkontaktsignal eine Sicherheitsstufe der jeweils zugeordneten Meldung (S2) auszuwerten.

9. Fernsteuereinheit (F) nach einem der vorangehenden auf die Fernsteuereinheit (F) gerichteten Patentansprüche, die eine Fernsteuersignaleinheit umfasst, die dazu ausgebildet ist, während der Ausführung der Bearbeitungsprozedur einen Fernbearbeitungszustand durch Ausgabe eines Fernbearbeitungssignals auf dem Anwendungsgerät (AG) zu kennzeichnen.

10. Fernsteuereinheit (F) nach einem der vorangehenden auf die Fernsteuereinheit (F) gerichteten Patentansprüche, die eine Prüfeinheit umfasst, die dazu ausgebildet ist, zu bestimmen, für welche medizinischen Geräte (DG) das jeweilige Anwendungsgerät (AG), dem die Fernsteuereinheit (F) zugeordnet ist, zulässig aktivierbar ist.

11. Medizinisches Anwendungsgerät (AG) mit einer Fernsteuereinheit (F) nach dem vorangehenden auf die Fernsteuereinheit (F) gerichteten Patentanspruch.

## Claims

1. A method of controlling a medical apparatus (DG) by processing messages (S2) on a remote control unit (F) of an application apparatus (AG), wherein the application apparatus (AG) is remote from but in visual contact with the medical apparatus (DG), and wherein the messages relate to the operation of the medical apparatus (DG), comprising the steps of:
- configuration of a safety criterion, where the safety criterion is defined specifically for each medical apparatus (DG),
- detecting the presence of a user in front of the application apparatus (AG) by means of an identification device (ID) provided on or at the application apparatus (AG); and;
- detecting (a) an activation signal on the application apparatus (AG);
- receiving (b) the respective message (S2) of the medical apparatus (DG) on the application apparatus (AG);
- by means of the identification device (ID): checking (c) the safety criterion with an evaluation of a visual contact signal representing a condition whether there is visual contact between the application apparatus (AG) on which the user assigned to it is working and the medical apparatus (DG) and/or a patient connected to the medical apparatus (DG), wherein the visual contact signal is evaluated by evaluating an RFID chip, an NFC module, a camera, by radio triangulation or by evaluating digital site plans
and in case of positive checking result:
∘ Providing a processing procedure for remotely processing (d) the respective message (S2) on the application apparatus (AG) as long as the safety criterion is met.

2. The method according to claim 1, wherein the safety criterion is configured in a definition phase.

3. Method according to any of the preceding claims, in which the checking of the safety criterion is carried out by evaluating a safety level of the associated message (S2) in addition to the visual contact signal.

4. A method according to any of the preceding claims, wherein in a definition phase for each medical apparatus (DG) a selection of target application devices is configured, on each of which a processing procedure is provided for remote processing, which is executable if the safety criterion is met.

5. A method according to any of the preceding claims, wherein during execution of the processing procedure, a remote processing state is indicated by the output of a remote processing signal on the application apparatus (AG).

6. A remote control unit (F) for a medical application apparatus (AG) for remotely controlling a medical apparatus (DG), the application apparatus (AG) being remote from but in visual contact with the medical apparatus (DG), by processing messages (S2) concerning the operation of the medical apparatus (DG) with:
- an input interface (E) which is intended to detect an activation signal (S2) and which is further intended to receive the respective message (S2) of the medical apparatus (DG), and comprising
- an identification device (ID) provided on or on the application apparatus (AG) and which is configured,
∘ to detect the presence of a user in front of the application apparatus (AG) and
∘ to define a safety criterion specific to each medical apparatus (DG), and
∘ to check the safety criterion while automatically evaluating a visual contact signal, the visual contact signal representing a condition of whether there is visual contact between the application apparatus (AG) on which the user assigned thereto is working and the medical apparatus (DG) and/or a patient connected to the medical apparatus (DG); and
∘ wherein the identification device (ID) for evaluating the visual contact signal evaluates an RFID chip, an NFC module, a camera and/or digital site plans and/or comprises a processor component intended for carrying out radio triangulation;
- wherein the remote control unit (F) is configured to execute a processing procedure for remotely processing the respective message (S2) in case of a positive check result as long as the safety criterion is met.

7. Remote control unit (F) according to one of the preceding patent claims directed to the remote control unit (F), in which the identification device (ID) is configured in a definition phase to define the safety criterion or to read in the safety criterion from a connected database.

8. Remote control unit (F) according to any of the preceding claims directed to the remote control unit (F), in which the identification device (ID) is configured, in the context of checking the safety criterion, to evaluate a safety level of the respectively assigned message (S2) for the visual contact signal.

9. A remote control unit (F) according to any of the preceding claims directed to the remote control unit (F), comprising a remote control signal unit adapted to indicate a remote processing state by outputting a remote processing signal on the application apparatus (AG) during execution of the processing procedure.

10. Remote control unit (F) according to any of the preceding claims directed to the remote control unit (F), comprising a test unit adapted to determine for which medical apparatus (DG) the respective application apparatus (AG) to which the remote control unit (F) is assigned is permissibly activatable.

11. Medical application apparatus (AG) comprising a remote control unit (F) according to the preceding patent claim directed to the remote control unit (F).

## Revendications

1. Procédé de commande d'un dispositif médical (DG) en traitant des messages (S2) sur une unité de commande à distance (F) d'un dispositif d'application (AG), dans lequel le dispositif d'application (AG) est éloigné du dispositif médical (DG) mais en contact visuel avec celui-ci et dans lequel les messages concernent le fonctionnement du dispositif médical (DG), comprenant les étapes consistant à :
- Configuration d'un critère de sécurité, où le critère de sécurité est défini spécifiquement pour chaque dispositif médical (DG),
- détecter la présence d'un utilisateur devant le dispositif d'application (AG) au moyen d'un moyen d'identification (ID) prévu sur ou au niveau du dispositif d'application (AG); et ;
- Détecter (a) un signal d'activation sur le dispositif d'application (AG) ;
- Réception (b) du message respectif (S2) du dispositif médical (DG) sur le dispositif d'application (AG) ;
- au moyen du dispositif d'identification (ID) : vérification (c) du critère de sécurité avec une évaluation d'un signal de contact visuel représentant un état indiquant s'il existe un contact visuel entre le dispositif d'application (AG) sur lequel travaille l'utilisateur qui lui a été attribué et le dispositif médical (DG) et/ou un patient connecté au dispositif médical (DG), dans lequel le signal de contact visuel est évalué en évaluant une puce RFID, un module NFC, une caméra, par triangulation radio ou en évaluant des plans de position numérique et en cas de résultat positif de la vérification :
∘ Fournir une procédure de traitement pour traiter à distance (d) le message respectif (S2) sur le dispositif d'application (AG) aussi longtemps que le critère de sécurité est satisfait.

2. Procédé de la revendication 1, dans lequel le critère de sécurité est configuré dans une phase de définition.

3. Procédé selon l'une quelconque des revendications précédentes, dans lequel la vérification du critère de sécurité est effectuée en évaluant, en plus du signal visuel de contact, un niveau de sécurité du message associé (S2).

4. Procédé selon l'une quelconque des revendications précédentes, dans lequel, dans une phase de définition, pour chaque dispositif médical (DG), on configure une sélection de dispositifs d'application cibles, sur chacun desquels est prévue une procédure de traitement à distance, exécutable si le critère de sécurité est satisfait.

5. Procédé selon l'une quelconque des revendications précédentes, dans lequel pendant l'exécution de la procédure d'usinage, un état de télé-usinage est indiqué par la sortie d'un signal de télé-usinage sur le dispositif d'application (AG).

6. Unité de commande à distance (F) pour un dispositif d'application médical (AG) pour commander à distance un dispositif médical (DG), le dispositif d'application (AG) étant éloigné du dispositif médical (DG) mais en contact visuel avec celui-ci, en traitant des messages (S2) relatifs au fonctionnement du dispositif médical (DG) avec:
- une interface d'entrée (E) destinée à détecter un signal d'activation (S2) et destinée en outre à recevoir le message respectif (S2) du dispositif médical (DG), et comprenant
- un dispositif d'identification (ID) prévu sur ou dans le dispositif d'application (AG) et destiné à,
∘ détecter la présence d'un utilisateur devant le dispositif d'application (AG) et
∘ définir un critère de sécurité spécifique à chaque dispositif médical (DG), et
∘ vérifier le critère de sécurité tout en évaluant automatiquement un signal de contact visuel, le signal de contact visuel représentant une condition indiquant s'il y a un contact visuel entre le dispositif d'application (AG) sur lequel l'utilisateur qui y est affecté travaille et le dispositif médical (DG) et/ou un patient connecté au dispositif médical (DG) ; et
∘ dans lequel le dispositif d'identification (ID) pour l'évaluation du signal de contact visuel évalue une puce RFID, un module NFC, une caméra et/ou des plans de site numériques et/ou comprend un composant processeur qui est destiné à effectuer une triangulation radio ;
- dans lequel l'unité de commande à distance (F) est adaptée pour exécuter une procédure de traitement pour traiter à distance le message respectif (S2) en cas de résultat de vérification positif tant que le critère de sécurité est rempli.

7. Unité de commande à distance (F) selon l'une des revendications de brevet précédentes, dirigée vers l'unité de télécommande (F), dans laquelle le dispositif d'identification (ID) est conçu, dans une phase de définition, pour définir le critère de sécurité ou pour lire le critère de sécurité dans une base de données connectée.

8. Unité de commande à distance (F) selon l'une des revendications de brevet précédentes, dirigée vers l'unité de commande à distance (F), dans laquelle le dispositif d'identification (ID) est conçu, dans le cadre de la vérification du critère de sécurité, pour évaluer un niveau de sécurité du message (S2) respectivement attribué pour le signal de contact visuel.

9. Unité de commande à distance (F) selon l'une quelconque des revendications précédentes, dirigée vers l'unité de commande à distance (F), comprenant une unité de signal de commande à distance adaptée pour indiquer un état de traitement à distance en émettant un signal de traitement à distance sur l'appareil d'application (AG) pendant l'exécution de la procédure de traitement.

10. Unité de commande à distance (F) selon l'une des revendications de brevet précédentes, dirigée vers l'unité de commande à distance (F), comprenant une unité de test adaptée pour déterminer pour quels dispositifs médicaux (DG) le dispositif d'application respectif (AG) auquel l'unité de commande à distance (F) est associée est activable de manière autorisée.

11. Dispositif d'application médicale (AG) comprenant une unité de commande à distance (F) selon la revendication de brevet précédente dirigée vers l'unité de commande à distance (F).
